# EUROPÄISCHE PATENTSCHRIFT

(11) **EP 1 042 449 B1**
(45) Veröffentlichungstag und Bekanntmachung des Hinweises auf die Patenterteilung: **04.05.2005**
(21) Anmeldenummer: 98960856.7
(22) Anmeldetag: 21.12.1998
(51) Int. Cl.: C12N 1/20, A23K 1/16, C12R 1/01

(54) **MIKROORGANISMUS, VERFAHREN ZUR GEWINNUNG DESSELBEN SOWIE FUTTERMITTELZUSATZ**
MICROORGANISM, METHOD FOR OBTAINING SAME AND FEED ADDITIVE
MICRO-ORGANISME, PROCEDE PERMETTANT DE LE PREPARER ET ADDITIF POUR SUBSTANCES FOURRAGERES

(30) Priorität: 30.12.1997 AT 220497
(43) Veröffentlichungstag der Anmeldung: 11.10.2000
(73) Patentinhaber: Erber Aktiengesellschaft, 3130 Herzogenburg (AT)
(72) Erfinder: BINDER, Eva-Maria, A-3430 Tulln (AT); BINDER, Johann, A-3430 Tulln (AT)
(74) Vertreter: Cunow, Gerda
(86) Internationale Anmeldenummer: PCT/AT1998/000316
(87) Internationale Veröffentlichungsnummer: WO 1999/035240

(56) Entgegenhaltungen:
- DE-A- 4 205 196
- BINDER, J. ET AL.: "Screening for deoxynivalenol-detoxifying anaerobic rumen microorganisms." CEREAL RESEARCH COMMUNICATIONS, (1997) VOL. 25, NO. 3 PART 1, PP. 343-346. ISSN: 0133-3720., XP002103921
- MATSUSHIMA, TOKUO (1) ET AL: "Deacetylation of diacetoxyscirpenol to 15-acetoxyscirpenol by rumen bacteria." JOURNAL OF GENERAL AND APPLIED MICROBIOLOGY, (1996) VOL. 42, NO. 3, PP. 225-234. ISSN: 0022-1260., XP002103922
- BOUTIBONNES P.: "Properties of a cell-line of bacillus thuringiensis (Berliner) resistant to the mycotoxin zearaleone." IRCS MEDICAL SCIENCE, (1980) 8/8 (527-528). CODEN: IRLCDZ, XP002103923

### SEQUENZPROTOKOLL

<110> Erber Aktiengesellschaft
<120> Mikroorganismus, Verfahren zur Gewinnung desselben sowie Futtermittelzusatz
<130> 1570
<140> PCT/AT98/00316
   <141> 1998-12-21
<150> A2204/97
   <151> 1997-12-30
<160> 1
<170> PatentIn Ver. 2.1
<210> 1
   <211> 1477
   <212> DNA
   <213> Enterococcus casseliflavus
<220>
   <223> The microorganism belongs to Genus Eubacterium, called Eubacterum sp., strain: Enterococcus casseliflavus, 16S RNA-sequence, Deposition Nos.: DSM 11798 and DSM 11799
<400> 1
   cctggctcag gatgaacgct ggcggcgtgc ttaacacatg caagtcgaac ggataacccg 60
   cctccgggcg gttatagagt ggcgaacggg tgagtaacac gtgaccaacc tacctcccac 120
   tccgggataa cccagggaaa cctgcgctaa taccggatac tccggggccc ccgcatgggg 180
   gcgccgggaa agccccgacg gtgggagatg gggtcgcggc ctattaggta gtcggcgggg 240
   taacggccca ccgagcccgc gataggtagc cgggttgaga gaccgatcgg ccacattggg 300
   actgagatac ggcccagact cctacgggag gcagcagtgg ggaattttgc gcaatggggg 360
   aaaccctgac gcagcaacgc cgcgtgcggg acgaaggcct tcgggttgta aaccgctttc 420
   agcagggaag aagttgacgg tacctgcaga agaagctccg gctaactacg tgccagcagc 480
   cgcggtaata cgtagggagc gagcgttatc cggatttatt gggcgtaaag cgcgcgtagg 540
   cgggcgctta agcggaatct ctaatctgag ggctcaaccc ccagccggat tccgaactgg 600
   gcgcctcgag ttcggtagag gaagacggaa ttcccagtgt agcggtgaaa tgcgcagata 660
   ttgggaagaa caccgatggc gaaggcagtc ttctgggccg taactgacgc tgaggtgcga 720
   aagctagggg agcgaacagg attagatacc ctggtagtcc tagccgtaaa cgatgggcac 780
   taggtgtggg ggggaatgcc cctccgtgcc gcagctaacg cattaagtgc cccgcctggg 840
   gagtacggcc gcaaggctaa aactcaaagg aattgacggg ggcccgcaca agcagcggag 900
   catgtggctt aattcgaagc aacgcgaaga accttaccag ggcttgacat gcaggtgaag 960
   cggcggaaac gccgtggccg agaggagcct gcacaggtgg tgcatggctg tcgtcagctc 1020
   gtgtcgtgag atgttgggtt aagtcccgca acgagcgcaa cccctgtcgt atgttgccat 1080
   cattcagttg gggactcgta cgagactgcc ggcgtcaagc cggaggaagg tggggacgac 1140
   gtcaagtcat catgcccttt atgccctggg ctgcacacgt gctacaatgg ccggtacaac 1200
   gggctgcgag ccagcgatgg cgagcgaatc cctcaaaacc ggtcccagtt cggatcggag 1260
   gctgcaaccc gcctccgtga agtcggagtt gctagtaatc gcggatcagc atgccgcggt 1320
   gaatacgttc ccgggccttg tacacaccgc ccgtcacacc acccgagttg tctgcacccg 1380
   aagtcgacgg cccaacccgc gaggggggag tcgccgaagg tgtggggagt aaggggggtg 1440
   aagtcgtaac aaggtagccg taccggaagg tgcggct                         1477

## Beschreibung

Die vorliegende Erfindung bezieht sich auf einen Mikroorganismus vom Genus Eubacterium, welcher in Reinkultur zur Detoxifizierung von Trichothecenen geeignet ist, sowie auf ein Verfahren zur Isolierung desselben, seine Produktion und Formulierung und seine Verwendung und einen den Mikroorganismus enthaltenden Futtermittelzusatz.

Trichothecene, welche zur Klasse der Mykotoxine gehören, sind in zahlreichen Futtermitteln von Tieren enthalten, wobei sie üblicherweise über auf Getreiden oder Gräsern befindliche Schimmelpilze in die Futtermittel eingeschleppt werden. Durch die unerwünschte Verabreichung von Mykotoxinen, insbesondere Trichothecenen, an Tiere wird sowohl deren Leistungsfähigkeit als auch beispielsweise das Wachstum der Tiere gehemmt, wobei es neben einer Schädigung der Gesundheit der Tiere auch zu einem erhöhten Futtermittelverbrauch bei gleichzeitig schlechterer Futtermittelverwertungsrate kommt. Zur Beseitigung der negativen Wirkungen von Mykotoxinen sind bereits zahlreiche Verfahren zum Binden bzw. Adsorbieren dieser Toxine bekannt geworden.

So ist beispielsweise in der WO 91/13555 ein Futtermittelzusatz sowie ein Verfahren zur Inaktivierung von Mykotoxinen beschrieben, wobei dem Futter Teilchen eines Phyllosilikatminerals zugesetzt werden, um die Mykotoxine zu inaktivieren. Zur Verstärkung der Wirkung dieser Phyllosilikate sind die Teilchen mit einem Sequestrierungsmittel beschichtet, um die Wirkung zu beschleunigen. Weiters ist beispielsweise aus der WO 92/05706 ein Futtermittel bekannt geworden, in welchem Montmorillonit-Ton als Futtermittelzusatz enthalten ist. Diese natürlichen Tonmineralien mit großer, innerer Oberflächen sollen aufgrund ihrer Porosität die Mykotoxine oberflächlich binden und auf diese Weise immobilisieren.

Weiters ist aus dem österreichischen Gebrauchsmuster AT-U 504 ein Futtermittelzusatz bekannt geworden, in welchem eine Enzymzubereitung verwendet wird, welche fähig ist, Epoxidasen und Laktonasen zu bilden und Mykotoxine chemisch sowohl im Futtermittel als auch im Magen-Darm-Trakt von Tieren abzubauen. Gemäß der AT-U 504 kann die Wirkung dieser Enzymzubereitung durch den Zusatz von Zeolithen und dgl. verstärkt werden.

Aus dem Dokument "Binder et al.", 1997, Cereal Res. Comm. 25:343-346, ist eine Untersuchung betreffend Deoxynivalenol detoxifizierende, anaerobe Mikroorganismen bekannt geworden. Bei diesen Untersuchungen konnten Mischkulturen von Mikroorganismen gefunden werden, welche Deoxynivalenol reduktiv zu dem Deepoxymetaboliten umwandeln, wobei eine Charakterisierung dieser Mischkultur nicht gelungen ist.

Die vorliegende Erfindung zielt nun darauf ab, einen speziellen Mikroorganismus bzw. eine definierte Mischkultur isoliert aus einem natürlichen Habitat zur Verfügung zu stellen, mit welchem es gelingt Mykotoxine, insbesondere Trichothecene, gezielt durch biochemischen Abbau in physiologisch unbedenkliche und insbesondere in der Tierzucht unbedenkliche Substanzen umzuwandeln.

Zur Lösung dieser Aufgabe wurde ein Mikroorganismus vom Genus Eubacterium isoliert, welcher in Reinkultur, DSM 11798 zur Detoxifizierung von Trichothecenen geeignet ist.

Der Mikroorganismus vom Genus Eubacterium, welcher aufgrund seiner Zugehörigkeit zum Genus Eubacterium auch Eubacterium sp. genannt wird und welcher in Reinkultur bei der Sammlung deutscher Mikroorganismen unter der Nummer DSM 11798 hinterlegt wurde, ist erfindungsgemäß insbesondere zur Detoxifizierung von insbesondere Deoxynivalenol (DON), T-2 Toxin, HT-2 Toxin, Nivalenol, Monoacetoxyscirpenol, Diacetoxyscirpenol, Trichodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol, Culmorin geeignet. Der Mikroorganismus gemäß der Erfindung detoxifiziert die Trichothecene durch reduktive Biotransformation der im Molekül enthaltenen Epoxidgruppe, welche Epoxidgruppe für die Toxizität der Mykotoxine, insbesondere Trichothecene verantwortlich ist. Der Abbau der Epoxidgruppe erfolgt bei den Trichothecenen entsprechend der nachfolgenden Formel durch reduktive Spaltung des toxischen 12,13-Epoxyringes:

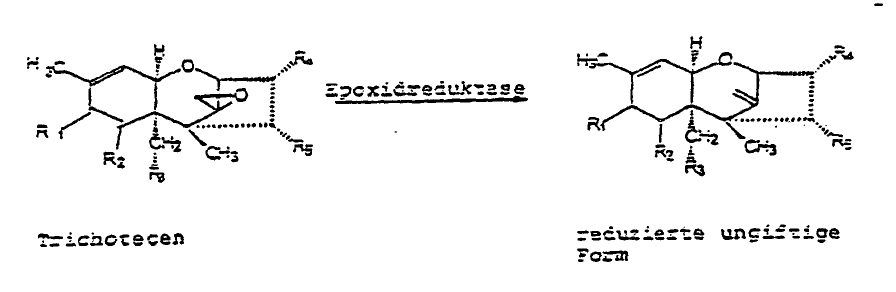

Die Morphologie des erfindungsgemäßen Mikroorganismus zeigt bevorzugt, daß er ein anaerobes Gram-positives, stäbchenförmiges, insbesondere 0,1 bis 3 µm langes, nicht-sporenbildendes Bakterium ist, welches sowohl einzeln, paarweise oder in langen Ketten, insbesondere bis zu etwa 150 µm, auftritt. Die phylogenetische Analyse des Mikroorganismus gemäß der Erfindung hat insbesondere eine 16S-RNA-Sequenz, nämlich ergeben, wobei die Sequenzdaten des Mikroorganismus mit bekannten 16S-RNA-Gensequenzen von repräsentativen Mikroorganismen, die der Domäne Bakteria angehören, verglichen wurden. Diese Vergleichsanalyse ergab eine größte Übereinstimmung mit Bakterien des Genus Eubacterium. Es konnte jedoch keine ausreichend übereinstimmende Gensequenz mit einem bekannten Mikroorganismus aufgefunden werden, woraus sich ergibt, daß der Mikroorganismus gemäß der Erfindung einen bis dato noch nicht isolierten und klassifizierten Mikroorganismus innerhalb des Genus Eubacterium darstellt. Auch physiologische Untersuchungen, wie z.B. Gärspektren, Reduktion von Nitrat zu Nitrit, ergaben eindeutig die Zugehörigkeit zum Genus Eubacterium.

Eine weitere Aufgabe der vorliegenden Erfindung ist ein Verfahren zur Gewinnung einer Reinkultur des Mikroorganismus DSM 11798 unter Verwendung einer Mischkultur mit dem Stamm Enterococcus casseliflavus, DSM 11799, zur Verfügung zu stellen, wobei insbesondere auf eine Ausbeuteoptimierung sowohl im wirtschaftlichen als auch im quantitativen Hinblick abgezielt wurde.

Zur Lösung dieser Aufgabe wird das erfindungsgemäße Verfahren so geführt, daß zur Gewinnung einer Reinkultur des obengenannten Mikroorganismus vom Genus Eubacterium, DSM 11798, eine Mischkultur DSM 11799 aus dem Mikroorganismus und Enterococcus casseliflavus aus Rinderpansen durch wenigstens zweimaliges Anzüchten bzw. Fermentieren in Verdünnungsreihen und anaeroben Kultivierungsbedingungen gewonnen wird. Zur Gewinnung der Reinkultur des Mikroorganismus gemäß der Erfindung hat sich ein wenigstens zweimaliges Anzüchten und/oder Fermentieren in Verdünnungsreihen als günstig erwiesen, da auf diese Weise eine gesicherte Reinheit der gewünschten Produkte und insbesondere eine Abtrennung von störenden Nebenprodukten bzw. Verunreinigungen mit unerwünschten Mikroorganismen erzielt werden kann. Zur Aufrechterhaltung der anaeroben Bedingungen wurde die Anzucht und/oder Fermentation gemäß der Erfindung bevorzugt in einer Gasatomsphäre aus H₂ und CO₂ vorgenommen, wobei besonders bevorzugt die Gasatmosphäre mit einem Verhältnis H₂:CO₂ von 10:90 bis 90:10, insbesondere etwa 80:20, gewählt wird. Für das Wachstum des Mikroorganismus gemäß der Erfindung sind anaerobe Bedingungen bei niedrigem Redoxpotential von Bedeutung, wobei überraschend ein ausreichend rasches Wachstum nur in Anwesenheit von H₂ erzielt werden kann.

Ein noch rascheres Wachstum des Mikroorganismus gemäß der Erfindung kann dadurch erzielt werden, daß die Anzucht und/oder Fermentation bei einem Überdruck von 0,2 bis 3 bar, insbesondere 0,5 bis 1 bar, durchgeführt wird, wie dies einer weiteren bevorzugten Ausführungsform entspricht. Ein weiter verbessertes Wachstum des erfindungsgemäßen Mikroorganismus DSM 11798 konnte dadurch erzielt werden, daß bevorzugt die Anzucht und/oder Fermentation bei einer Temperatur von 35 bis 42 °C, insbesondere etwa 37 °C, durchgeführt wird. Das pH-Optimium für die Anzucht bzw. Fermentation lag bei dem erfindungsgemäßen Verfahren bevorzugt bei einem pH-Wert zwischen 6 und 8 und insbesondere zwischen 7 und 7,5. Unter diesen Bedingungen ist es möglich, sowohl eine Reinkultur des Mikroorganismus DSM 11798 als auch seiner oben beschriebenen Mischkultur (DSM 11799) in möglichst kurzer Zeit und unter Verwendung relativ weniger Verdünnungsreihen zu erhalten. Optimale Ergebnisse können mit dem erfindungsgemäßen Verfahren dadurch erzielt werden, daß bevorzugt die Anzucht und/oder Fermentation in einer Medienbereitung, enthaltend Komponenten gewählt aus: Arginin, Citrulin, Pepton, Hefeextrakt, Fettsäuremischung(en), Minerallösung(en), Glucose, Häminlösung, Menadion, Vitaminlösung, Spurenelementen, Reduktionsmittel, durchgeführt wird.

Die in einer Medienbereitung enthaltenen Komponenten sind hiebei teilweise austauschbar, wobei beispielsweise durch Zugabe von Glucose eine Verschiebung des Gleichgewichts in der Mischkultur in Richtung zu Enterococcus casseliflavus oder entsprechenden anderen anaeroben Mikroorganismen erzielt werden kann, wobei je nach Menge der zugesetzten Glucose das Verfahren gezielt gesteuert werden kann. Gemäß einem besonders bevorzugten Aspekt der Erfindung wird zu Beginn der Anzucht und/oder Fermentation 0,1 bis 0,5 Gew.-%, insbesondere 0,2 Gew.-%, Glucose zugesetzt. Durch einen Zusatz von 0,1 bis 0,5 Gew.-% Glucose wird zu Beginn der Anzucht und/oder Fermentation das Wachstum von Enterococcus casseliflavus gefördert, was zu einem Absinken des Redoxpotentials führt. Durch Absenken des Redoxpotentials wurden optimale Wachstumsbedingungen für den Mikroorganismus gemäß der Erfindung geschaffen, so daß durch eine gezielte Glucosezugabe beispielsweise auf Chemikalien, wie Cystein, in der Medienbereitung verzichtet werden kann.

Um die Detoxifizierung von Mykotoxinen, insbesondere Trichothecenen, auf andere vorteilhafte Wirkung mit dem erfindungsgemäßen Mikroorganismus zu erzielen, können erfindungsgemäß bevorzugt auch Enzympräparationen des aktiven, Trichothecen-detoxifizierenden Mikroorganismus und/oder andere anaerobe Mikroorganismen zugesetzt werden.

Zur Erzielung einer Reinkultur des Mikroorganismus DSM 11798 wird das erfindungsgemäße Verfahren weiter so geführt, daß aus der Anzucht- bzw. Fermentationslösung DSM 11799 die Reinkultur des Mikroorganismus DSM 11798 durch wenigstens zwei weitere Verdünnungsreihen in der Medienbereitung, insbesondere unter Zusatz von L-Arginin als Wachstumsstimulator, gewonnen wird. Indem zwei weitere Verdünnungsreihen in der Medienbereitung aus der Anzucht- bzw. Fermentationslösung durchgeführt werden, kann der Mikroorganismus DMS 11798 vollständig rein aus der Mischkultur mit Enterococcus casseliflavus gewonnen werden, wobei der Zusatz des Wachstumsstimulators L-Arginin das Gleichgewicht vorteilhafterweise in Richtung der Reinkultur des Mikroorganismus verschiebt. Hiebei wird, je höher die Konzentration von L-Arginin ist, das Wachstum des Bakteriums gemäß der Erfindung gefördert.

Um das Redoxpotential der Medienbereitung weiter abzusenken, wird erfindungsgemäß bevorzugt so vorgegangen, daß zur Fermentation der Reinkultur der Medienbereitung 1 bis 4 Gew.-% eines Reduktionsmittels, insbesondere einer Mischung von Cystein/Natriumsulfid/Natriumcarbonatlösung, zugesetzt wird. Besonders aus Wirtschaftlichkeitsgründen wird erfindungsgemäß der Zusatz des Reduktionsmittels so niedrig wie möglich gehalten, wobei sich im Zuge von Vergleichsversuchen ergeben hat, daß eine Steigerung der Konzentration des Reduktionsmittels über 4 Gew.-% hinaus keine weitere Beschleunigung des Wachstums des Mikroorganismus bewirkt.

Zur Erzielung eines lagerfähigen Fertigproduktes wird das erfindungsgemäße Verfahren bevorzugt so weitergeführt, daß die Anzucht- bzw. Fermentationslösung durch Konzentrieren, insbesondere Zentrifugieren oder Filtrieren und/oder Stabilisierung, insbesondere durch Gefrier- oder Sprühtrocknen oder Encapsulieren, aufgearbeitet wird. Hiebei wird beispielsweise in einem ersten Schritt die Anzucht- bzw. Fermentationslösung konzentriert, indem Flüssigkeit durch Zentrifugieren oder Filtrieren abgetrennt wird, und/oder unmittelbar aus der Fermentationslösung die Stabilisierung erfolgt, bevorzugt unter Zusatz eines Füllstoffes bzw. Trägermaterials, wie Aluminiumsilikate, Kieselgure, Kohlehydrate, Zuckeralkohole, Stärken, Milch und Molkepulver, Proteinhydrolysate, Hefen, PVPP. Durch Zusatz dieser Träger oder Füllstoffe kann in dem nachfolgenden Stabilisierungsschritt, insbesondere dem Gefriertrocken-, Sprühtrocken-, Encapsulierungs- bzw. Pelletierungsschritt ein Festprodukt erhalten werden, in welchem die Reinkultur des Mikroorganismus DSM 11798 direkt auf einem Träger abgeschieden ist, wodurch ein besonders leicht handhabbares und lagerfähiges sowie stoffwechselförderliches Produkt erzielt wird. Durch Abscheiden des Mikroorganismus auf einer Substanz mit großer innerer Oberfläche, wie Tonerden, Aluminiumsilikaten, Zeolithen und dgl., wird weiters der erfindungsgemäß beabsichtigte Abbau von Trichothecenen erleichtert, da diese physikalisch an die Substanz mit großer innerer Oberfläche gebunden werden, worauf der biochemische Angriff mit dem erfindungsgemäßen Mikroorganismus deutlich erleichtert wird.

Erfindungsgemäß wird weiters der Mikroorganismus in Reinkultur (DSM 11798) zur Herstellung eines Futtermittelzusatzes verwendet. Eine erfindungsgemäß besonders bevorzugte Verwendung ergibt sich im wesentlichen dadurch, daß die Reinkultur (DSM 11798) als gefrier- oder sprühgetrocknetes und/oder encapsuliertes oder pelletiertes Immobilisat, gegebenenfalls unter Zusatz eines Trägermaterials, eingesetzt wird. Sowohl die Reinkultur des Mikroorganismus gemäß der Erfindung als auch das sprühgetrocknete Immobilisat können direkt als Futtermittelzusatz verwendet werden, wobei der Futtermittelzusatz bereits direkt bei der Herstellung dem Futtermittel beigemischt werden kann und/oder bei der Verfütterung an die Tiere in das Futtermittel sowohl in fester als auch in flüssiger Form eingemischt werden kann.

Um einen möglichst vollständigen Abbau bzw. chemische Umwandlung der Trichothecene in physiologisch unbedenkliche Substanzen zu erzielen, ist ein erfindungsgemäßer Futtermittelzusatz zur Inaktivierung von Trichothecenen in Futtermitteln bzw. im Verdauungstrakt von Tieren im wesentlichen dadurch gekennzeichnet, daß der Futtermittelzusatz eine Reinkultur eines erfindungsgemäßen Mikroorganismus oder einen erfindungsgemäß hergestellten Mikroorganismus in einer Menge von 10⁵ bis 10¹² Zellen/kg, insbesondere 10⁷ bis 10⁹ Zellen/kg, pro 1000 kg Futtermittel enthält. Durch einen Einsatz von 10⁵ bis 10¹² Zellen/kg, insbesondere 10⁷ bis 10⁹ Zellen/kg, des erfindungsgemäßen Mikroorganismus bzw. der erfindungsgemäßen Mischkultur aus dem Mikroorganismus und Enterococcus casseliflavus oder anderen anaeroben Mikroorganismen, insbesondere aus dem Genus Enterococcus, Streptococcus, Lactococcus, Bacillus oder Lactobacillus, die zur Detoxifizierung von Trichothecenen geeignet sind, ist es möglich, hohe Konzentrationen an Trichothecenen, insbesondere an Deoxynivalenol, T-2 Toxin, HT-2 Toxin, Nivalenol, Monoacetoxyscirpenol, Diacetoxyscirpenol, Trichodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol, Culmorin in Futtermitteln in chemisch unbedenkliche Substanzen, wie den Deepoxymethaboliten von Deoxynivalenol (DOM-1), umzuwandeln, so daß mit dem erfindungsgemäßen Futtermittelzusatz sowohl eine Leistungssteigerung der Tiere als auch aufgrund der verminderten Toxizität eine verbesserte Futterumwandlungsrate erzielt werden kann.

Um den biochemischen Abbau der Mykotoxine, insbesondere Trichothecene, weiter zu erleichtern, kann erfindungsgemäß bevorzugt in dem Futtermittelzusatz zusätzlich ein Trägermaterial und/oder Füllstoff in einer Menge von 0,5 bis 8 kg/1000 kg, insbesondere 0,7 bis 4 kg/1000 kg, des Futtermittels enthalten sein. Durch den Zusatz von Trägermaterialien und/oder Füllstoffen können gegebenenfalls die abzubauenden Mykotoxine als auch weitere Schadstoffe, welche in dem Futtermittel enthalten sein können, an die Substanzen physikalisch gebunden werden, wodurch sie für eine Verstoffwechslung nicht mehr zur Verfügung stehen.

Als Trägermaterial und/oder Füllstoff werden hiebei insbesondere Aluminiumsilikate, Kieselgure, Kohlehydrate, Zuckeralkohole, Stärke, Milch und Molkenpulver, Proteinhydrolysate, Hefen und/oder PVPP eingesetzt, wobei sich diese Trägermaterialien und/oder Füllstoffe für das Binden von Toxinen, insbesondere Trichotoxinen, als besonders vorteilhaft erwiesen haben.

Ein besonders bevorzugter Futtermittelzusatz ist dadurch gekennzeichnet, daß der Futtermittelzusatz aus einer Mischung aus 1 bis 65 Gew.-%, insbesondere 5 bis 50 Gew.-%, des sprüh- bzw. gefriergetrockneten Immobilates des Mikroorganismus und 99 bis 35 Gew.-%, insbesondere 95 bis 50 Gew.-%, Trägermaterial und/oder Füllstoff besteht. Derartige Futtermittelzusätze sind insbesondere zur Inaktivierung von Deoxynivalenol (DON), T-2 Toxin, HT-2 Toxin, Nivalenol, Monoacetoxyscirpenol, Diacetoxyscirpenol, Trichodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol, Culmorin sowohl im Futtermittel als auch im Verdauungstrakt von Tieren geeignet.

Die Erfindung wird nachfolgend anhand von Charakterisierungen des Mikroorganismus gemäß der Erfindung, seinen Wachstums- bzw. Aktivitätsbedingungen sowie der Bildung von Stoffwechselprodukten von Trichothecenen mit Hilfe des Mikroorganismus gemäß der Erfindung sowie anhand von Ausführungsbeispielen von Fütterungsversuchen weiter erläutert.

Der Mikroorganismus gemäß der Erfindung ist ein aktiver, Trichothecene transformierender Stamm, insbesondere Deoxynivalenol transformierender Stamm, und wird aus Rinderpansen durch wiederholte Anzucht in einem optimierten Nährmedium unter anaeroben Kultivierungsbedingungen, nämlich einer CO₂:H₂-Gasatmosphäre (20/80 v/v) und einem Überdruck von 0,5 bis 1,5 bar gewonnen. Als Medienbereitung wurden hiebei PYG- und PY-Medien verwendet, welche jeweils aus unterschiedlichen Konzentrationen aus zwei verschiedenen Minerallösungen, Menadionstammlösung, Häminlösung, Hefeextrakt, Pepton, bestanden, wobei dem PYG-Medium weiters Glucose zugesetzt war. Um das Redoxpotential abzusenken, wurden sowohl dem PYG- als auch dem PY-Medium 2 bis 4 Gew.-% einer Reduktionslösung aus Cystein/Na₂S/Na₂CO₃-Lösung zugesetzt und der pH wurde durch CO₂-Begasung auf einen Wert von 7 bis 7,5 eingestellt. Mit Hilfe dieser Medienbereitung gelingt es, eine Reinkultur des Mikroorganismus DSM 11798 zu gewinnen, indem mehrere Verdünnungsreihen durchgeführt werden. Das Wachstum des Mikroorganismus wird ausschließlich bei strikt anaeroben Bedingungen bei genügend niedrigem Redoxpotential und H₂-Anwesenheit erzielt. Das optimale Wachstum des Mikroorganismus kann bei etwa 37 °C erzielt werden, wobei jedoch auch zwischen 35 °C und 38 °C ein ausreichendes Wachstum des Mikroorganismus erzielt werden kann. Für die Kultivierung der Reinkultur des Mikroorganismus DSM 11798 wirkt L-Arginin in flüssigem Medium stimulierend.

Mit dem Mikroorganismus gemäß der Erfindung gelingt es, in Trichothecenen durch reduktive Spaltung des toxischen 12,13-Epoxyringes diese zu entgiften.

Das Reaktionsschema wird hiebei in der Folge anhand der Trichothecene allgemein sowie dem speziellen Trichothecen Deoxynivalenol gezeigt.

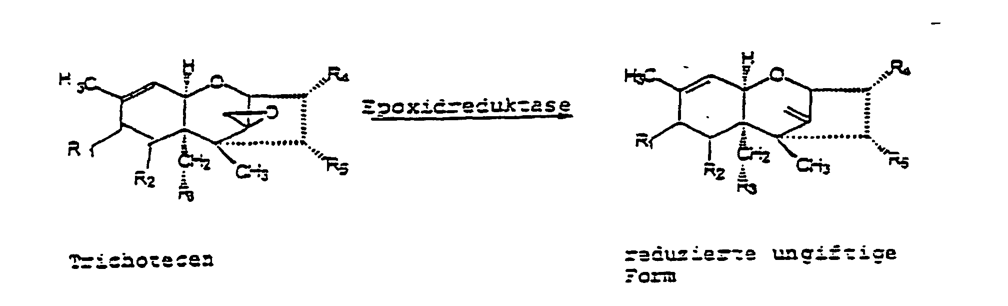

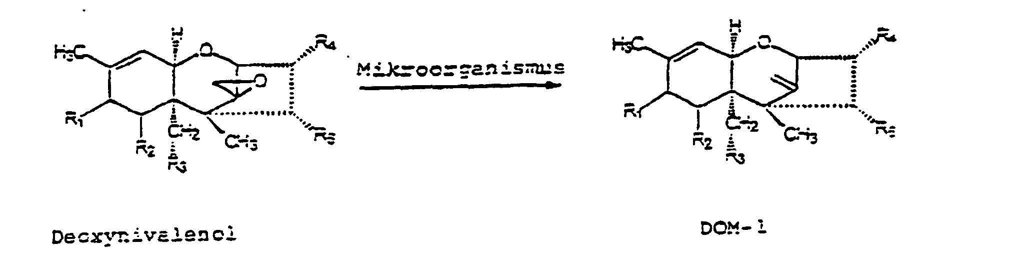

Zur Fermentation des erfindungsgemäßen Mikroorganismus DSM 11798 sowie in Cokultur mit anderen fakultativen und anaeroben Mikroorganismen sowie einer Mischkultur mit Enterococcus casseliflavus (DSM 11799) sollen in der Folge sowohl die Fermentationsbedingungen als auch verwendbare Fermentationsverfahren beispielhaft erläutert werden.

### Fermentationsbedingungen:

Fermentationstemperatur zwischen 35 und 42 °C, insbesondere etwa 37 °C.
pH-Wert Bereich für die Fermentation zwischen 6 und 8, insbesondere 7,0 - 7,5;
Redoxpotential: 0 - -350 mV, je nach Prozeßführung
Gasatmosphäre: H2/CO2 10:90 bis 90:10, insbesondere 80:20.
Fermentationsdruck: 0,2 - 3 bar, insbesondere 0,5 bis 1 bar.
Wesentliche Medienbestandteile: Arginin, Citrullin, Hefeextrakt, Peptone, Hemin und heminhältige Substanzen, niedere Fettsäuren, Minerallösung, Carbonatpuffer (Natriumcarbonat + CO2) gegebenenfalls Glucose, Spurenelementlösung, Vitaminlösung sowie Reduktionsmittel

Für die Fermentation können hiebei verschiedene Verfahrensführungen gewählt werden

### 1) Batch Fermentation der Reinkultur DSM 11798:

Verfahrensweise: Sterilisation des Mediums bei 121 °C und 1,5 bar oder Sterilfiltration. Abkühlung des Mediums auf Fermentationstemperatur 35 - 42 °C, insbesondere 37 °C, unter Begasung mit entkeimten CO2 und Zugabe von Natriumcarbonat und Reduktionsmittel. Die Begasung wird solange fortgeführt, bis ein pH-Wert von 6 - 8, insbesondere von 7 - 7,5, erreicht ist. Anschließend Zugabe von 1 - 10 % Inokolum, welches 24 - 48 vorkultiviert wurde, insbesondere 5 %. Fermentation bis zum Beginn der stationären Phase - Dauer etwa 20 - 50 h, je nach Substratkonzentration oder bis eine Keimzahl im Bereich von 10₁₃ - 10₁₆ erreicht ist.

Die Steuerung des Prozesses erfolgt im wesentlichen durch die Substratkonzentration.

### 2) Fed batch Fermentation der Reinkultur DSM 11798:

Verfahrensweise: Sterilisation, Pufferung, Reduktion und Inokulation des Mediums wie bei 1. Erhöhung der Biomasseausbeute durch diskontinuierliche oder kontinuierliche Dosierung von Substrat z. B. Arginin, Citrullin. Die Kultur wird durch Halten der Substratkonzentration auf höherem Niveau in der exponentiellen Wachstumsphase gehalten. Bei dieser Verfahrensführung ist eine Fermentationsdauer bis zu 60 h möglich.

Die Steuerung des Prozesses erfolgt durch die Substratdosierung und Fermentationsdauer (Anhäufung von Stoffwechselendprodukten).

### 3) Kontinuierliche Fermentation der Reinkultur DSM 11798:

Verfahrensweise: Sterilisation, Pufferung, Reduktion und Inokulation des Mediums wie bei 1. Batch-Fermentation bis zum Beginn der stationären Phase anschließend Umstellung auf kontinuierliche Fermentation durch Dosierung von steriler Nährlösung. Ablauf wird in Lagertank gesammelt und batchweise aufgearbeitet oder kontinuierlich sprühgetrocknet.

### 4) Fermentation der Reinkultur DSM 11798 in Cokultur mit anderen fakultativen und strikt anaeroben Mikroorganismen oder Fermentation der Cokultur DSM 11799

Beispiele für einsetzbare Cokulturen:

| | |
|---|---|
| H2-Produzenten | DSM 11798 + Butyrvibrio sp. DSM 11798 + Ruminococcus sp. |
| Probiotika | DSM 11798 + Enterococcus casseliflavus = DSM 11799 DSM 11798 + Streptococcus sp. (Enterococci, Milchsäure Streptococci, Anaerobe Streptococci) DSM 11798 + Leuconostoc sp. DSM 11798 + Pediococcus sp. DSM 11798 + Lactobacillus sp. DSM 11798 + Bifidobacterium sp. DSM 11798 + Bacillus sp. DSM 11798 + Megasphera sp. |
| Hefen | DSM 11798 + Saccaromyces sp. DSM 11798 + Klyveromyces sp. DSM 11798 + Candida sp. |

Einsatz von Coorganismen in der Fermentation dient einerseits zur Absenkung des Redoxpotentials in der Fermentation, zur Produktion von Wasserstoff für DSM 11798, als Schutzorganismus in der Aufarbeitung und Stabilisierung. Hiebei gelingt eine Minimierung von Keimzahlverlusten von DSM 11798 und sie dienen teilweise als zusätzlicher Leistungsförderer in der Tierproduktion.

### 4a) Batch-Fermentation in Cokultur:

I) Vorkultivierung des Coorganismus auf Kohlehydrate-hältigem Medium. Es wird das oben beschriebene Medium, welches jedoch kein Reduktionsmittel aber dafür zusätzlich Kohlehydrate enthält, zur Senkung des Redoxpotentials verwendet. Anschließend Inaktivierung des Coorganismus und Inokulation von DSM 11798.

II) Gleichzeitige Inokulation des Coorganismus und DSM 11798 und Zusatz von 0,1 - 1 % Kohlehydrate ins Medium. Es wird das oben beschriebene Medium, welches jedoch kein Reduktionsmittel aber dafür zusätzlich Kohlehydrate enthält, verwendet. Das Wachstum des Coorganismus wird gefördert - rasches Absinken des Redoxpotentials - DSM 11798 beginnt aufgrund der idealen Wachstumsbedingungen zu wachsen.

III) In Kombination mit I + II: Zu Fermentationsende Zugabe von Kohlenhydraten zur Auffermentation des Coorganismus. Dies führt zu einem Schutzeffekt (Sauerstoff) bei der Aufarbeitung und Stabilisierung aufgrund der erhöhten Biomasseausbeute.

### 4b) Fed batch Fermentation in Cokultur:

*)batch Phase entsprechend 4aI, anschließend kontinuierliche/diskontinuierliche Dosierung von Substrat (Arginin, Citrullin) entsprechend 2.

**) batch Phase entsprechend 4aI, anschließend kontinuierliche/diskontinuierliche Dosierung einer Substratkombination (Arginin/Kohlehydrate oder Citrullin/Kohlehydrate).

### 4c) kontinuierliche Fermentation der Cokultur:

batch Phase entsprechend 4aI, anschließend Umstellung auf kontinuierliche Fermentation durch Dosierung einer Arginin/Kohlehydrat bzw. Citrullin/Kohlehydrat-hältigen Nährlösung. Aufarbeitung wie bei 3.

### Aufarbeitung der Fermentationsprodukte:

1) Konzentrierung durch Membranfiltrationsverfahren (Ultrafiltration, Mikrofiltration) oder Zentrifugation. Anschließend Sprühtrocknung oder Lyophilisation mit oder ohne organische und/oder anorganische Trägermaterialien.
2) Direkte Sprühtrocknung oder Lyophilisation mit oder ohne organische und/oder anorganische Trägermaterialien.
3) Kontinuierliche Sprühtrocknung der Fermentationsbrühe mit oder ohne organische und/oder anorganische Trägermaterialien.
4) Encapsulierung oder Pelletierung in Kombination mit 1, 2 oder 3.

Zur Überprüfung der Aktivität des DON-biotransfomierenden Stammes (DSM 11798) im Darmmilieu wurde ein *In vitro* Modell mit Schweinedarm entwickelt.

Hiebei wurde in einem 1. Versuch ein *In vitro* Modell mit Darminhalten in Puffer unter Zusatz von Lyophilisat entwickelt.

Dünndarm und Dickdarm von schlachtfrischem Schwein wurde unter CO₂-Atmosphäre gehalten. Die Entleerung der Inhalte der Abschnitte vorderer, mittlerer, hinterer Dünndarm und Dickdarm in einzelne Gefäße wurde unter CO₂ Atmosphäre vorgenommen.

20 ml anaerober Puffer + 1 g Darminhalt (CO₂ begast) + DON wurden bei 37 °C unter CO₂- bzw. H₂/CO₂- Atmosphäre inkubiert.

Hiebei wurde festgestellt, daß im vorderen Dünndarmabschnitt ein deutlich positiver Effekt bei Zusatz von aktivem Lyophilisat zu bemerken ist. Wesentlich ist hiebei insbesondere die Tatsache, daß die deutliche Aktivität auch unter reiner CO₂-Begasung festzustellen war.

In einem 2. *In vitro* Versuch wurde mit ganzen Schweinedarmstückchen unter Zusatz aktiver Suspension gearbeitet.

Schweinedarmstücke (vordere, mittlerer, hinterer, Dickdarm, je 6 - 8 cm) wurde in anaeroben, reduzierten, präinkubierten Puffer (30 ml) bei 37 °C mit 200 ppm DON 24h inkubiert.

Diese modifizierte in vitro Test Variante hat den Vorteile, daß der physiologische Zustand des Darmes kaum beeinflußt wird, da sofort nach dem Schlachten der ganze Darm ins Labor transportiert wird und dort Stücke in gewünschter Länge abgebunden werden. Danach werden die Stücke abgetrennt und in Pufferlösung mit DON und aktiver Kultur inkubiert.

Die Ergebnisse sind eindeutig. Durch die aktive Kultur kann die Deepoxylierung von DON zu DOM-1 erzielt werden. Wesentlich ist, daß in sämtlichen Abschnitten des Darmes diese Aktivität nachgewiesen werden kann, wobei insbesondere im vorderen Darm die höchste Aktivität aufzufinden ist. Dies ist insoferne von Bedeutung, als der Großteil der Nahrungsresorption und somit auch die Freisetzung der Mykotoxine ebenfalls dort erfolgt.

Die Wirkung des erfindungsgemäßen Mikroorganismus sowohl in Reinkultur (DSM 11798) soll in der Folge anhand eines Laborprotokolls betreffend Hühnerzellkulturen sowie an Fütterungsbeispielen an Schweinen und Hühnern nachgewiesen werden.

Anhand eines Laborprotokolls mit Hühnerzellkulturen wird für den Mikroorganismus gezeigt, daß dieser in der Lage ist, Mykotoxine, insbesondere Trichothecene, und im speziellen Deoxynivalenol und T-2 Toxin chemisch abzubauen, insbesondere zu reduzieren und in physiologisch unbedenkliche Substanzen, im Falle von Deoxynivalenol in den Deepoxymetaboliten desselben, nämlich DOM-1, umzuwandeln.

Zur Kultivierung der Hühnerlymphozyten wurden folgende Bedingungen eingehalten:

| | | |
|---|---|---|
| verwendete Zellzahlen: 2x10⁶ Zellen/ml | | |
| Stimulation: ConA: 5 µg/ml | | |
| Mykotoxine: DON, DOM-1 und T2-Toxin, | | |
| Konzentrationsbereich | DON | 10 - 0,08 µg/ml |
| | DOM-1 | 232 - 1,81 µg/ml |
| | T2-Toxin | 30 - 0,234 ng/ml |

Gesamtinkubationszeit: 44 Stunden, davon 16 Stunden Labelingzeit bei Kultivierung im Brutschrank: 40 °C, 5 % CO₂, gesättigte Wasserdampfatmosphäre

Anhand eines Laborprotokolls mit Hühnerlymphocytenzellkulturen wird für die aktive Kultur gezeigt, daß diese in der Lage ist, Mykotoxine, insbesondere Trichothecene, biochemisch abzubauen, insbesondere zu reduzieren, und in physiologisch unbedenkliche Substanzen unbedenkliche Substanzen umzuwandeln. Dies wird im folgenden exemplarisch an DON und seinem Deepoxymetaboliten DOM-1, angeführt:

### Mikroskopische Kontrolle der Zellkultur:

Die Zellkultur mit den Hühnerlymphozyten wurde während der Kultivierung laufend mikroskopisch kontrolliert.

### Kontrolle nach 20 Stunden:

Die unstimulierten Zellen sind dicht und gleichmäßig verteilt, die Kontrollen mit ConA zeigen kräftige Stimulation und ausgeprägte Proliferationsherde.

DON: Es zeigen sich in allen Konzentrationstufen Proliferationsherde, wobei aber auffällt, daß in einem Konzentrationsbereich von 10 µg/ml-0,625 µg/ml deutliche Verkleinerungen der Proliferationsherde mit steigender Toxinkonzentration zu beobachten sind.

DOM: Proliferationsherde in allen Konzentrationsstufen ohne eine ersichtliche Änderung im Vergleich zur Kontrolle bis zur höchsten Konzentrationsstufe von 58 µg/ml.

Das bedeutet, daß schon nach 20 Stunden eine deutliche Beeinträchtigung der Zellaktivität im Toxinansatz ab einer Konzentration von 0,625 µg/ml vorliegt, während sich mit dem Deepoxymetaboliten selbst bei einer Konzentration von 58 µg/ml noch keine negativen Effekte auf die Zellkultur zeigten.

### Kontrolle nach 28 und nach 44 Stunden:

Die Kontroll-Ansätze (unstimuliert und ConA) sind unverändert.

Die Wirkung des DON auf die Zellkultur hat sich noch verstärkt. In jenen Reihen, in denen bereits nach 20 Stunden eine Veränderung festgestellt wurde, zeigten sich deutliche Schädigungen der Zellen.

Auch nach diesen Inkubationszeiten konnte selbst bei einer Konzentration des Deepoxymetaboliten von 58 µg/ml keine Beeinträchtigung der Zellaktivität gefunden werden.

In einem weiteren Versuch wurde der Effekt des erfindungsgemäßen Futtermittelzusatzes gegen eine Kontamination mit Trichothecenen in einem Kükenfutter gezeigt. Die verwendeten Parameter waren das Endgewicht, die Futteraufnahme und die Futterumwandlungsrate ebenso wie die Verluste an Küken. Die klinischen Symptome wurden ebenfalls aufgezeichnet.

Tiere: Küken der Rasse Cobb wurden vom ersten Lebenstag an untersucht. Der Versuch wurde mit drei Gruppen, umfassend 10.700, 10.900 und 15.700 Küken, durchgeführt. Den Hühnern wurde ein spezifisches Hühnerfutter ad libitum verabreicht.

Dosierung des Futtermittelzusatzes: Der Futtermittelzusatz war in einer Dosierung von 1 kg/t Kükenfutter enthalten, wobei lediglich die Versuchsgruppe den Futtermittelzusatz erhielt. Als Futtermittelzusatz wurde eine Reinkultur des Mikroorganismus (DSM 11798) eingesetzt.

### Keimzahl: 1.10⁹ Zellen/kg Fertigfutter

Der Versuchsgruppe und der positiven Vergleichsgruppe wurden in dem Futtermittel Trichothecene in einer Gesamtmenge von 750 ppb (500 ppb DON und 250 ppb T-2 Toxin) verabreicht.

Ergebnisse: Die nachfolgende Tabelle zeigt die Leistungsparameter aller drei Gruppen.

**Tabelle 1**

| | Positive Vergleichsgruppe | Negative Vergleichsgruppe | Versuchsgruppe |
|---|---|---|---|
| Anzahl der Tiere | 10.700 | 10.900 | 15.700 |
| mittleres Endgewicht in kg | 1,806 | 1,91 | 1,92 |
| mittlere Futteraufnahme in kg | 3,161 | 2,729 | 2,722 |
| Futterumwandlungsrate (FCR) | 1,75 | 1,43 | 1,42 |
| Verluste | 205 (1,92 %) | 175 (1,61 %) | 248 (1,58 %) |

Klinische Beobachtungen: Bei vielen Tieren der positiven Vergleichsgruppe wurden deutliche orale Irritationen ersichtlich.

Diskussion: Auch im vorliegenden Fall war der Futtermittelzusatz fähig, den negativen Einfluß der Trichothecene auf das Geflügel in bezug auf die Leistungsparameter und die klinischen Symptome vollständig zu kompensieren. Auch im Fall des Geflügels zeigt sich, daß Küken der Versuchsgruppe, welche neben Mykotoxinen auch den Mikroorganismus DSM 11798 erhielten, sogar ein geringfügig höheres mittleres Endgewicht als die negative Vergleichsgruppe aufwiesen und dies bei einer geringfügig niedrigeren mittleren Futtermittelaufnahme, wodurch sich sogar in bezug auf die negative Vergleichsgruppe etwas verbesserte Leistungsparameter ergeben. Dies zeigt bei Einsatz des erfindungsgemäßen Futtermittelzusatzes enthaltend den erfindungsgemäßen Mikroorganismus, daß nicht nur die negative Wirkung der Mykotoxine kompensiert wurde, sondern eine weitere Leistungssteigerung bei den Tieren, welche den Mikroorganismus DSM 11798 erhielten, erzielt werden konnte.

## Patentansprüche

1. Mikroorganismus vom Genus Eubacterium in Reinkultur, DSM 11798, geeignet zur Detoxifizierung von Trichothecenen durch reduktive Spaltung des 12,13-Expoxyrings derselben.

2. Mikroorganismus nach Anspruch 1, **dadurch gekennzeichnet, daß** er Deoxynivalenol (DON), T-2 Toxin, HT-2 Toxin, Nivalenol, Monoacetoxyscirpenol, Diacetoxyscirpenol, Trichodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol, Culmorin detoxifiziert.

3. Mikroorganismus nach Anspruch 1 oder 2, **dadurch gekennzeichnet, daß** der Mikroorganismus die Trichothecene durch reduktive Biotransformation der im Molekül enthaltenen Epoxidgruppe detoxifiziert.

4. Mikroorganismus nach Anspruch 1, 2 oder 3, **dadurch gekennzeichnet, daß** er ein anaerobes Gram-positives, stäbchenförmiges, nicht-sporenbildendes Bakterium ist, welches einzeln, paarweise oder in langen Ketten auftritt.

5. Mikroorganismus nach einem der Ansprüche 1 bis 4, **dadurch gekennzeichnet, daß** er eine 16S-RNA-Sequenz, nämlich aufweist.

6. Verfahren zur Gewinnung einer Kultur des Mikroorganismus nach einem der Ansprüche 1 bis 5, **dadurch gekennzeichnet, daß** zuerst eine Mischkultur DSM 11799 aus dem Mikroorganismus und Enterococcus casseliflavus aus Rinderpansen durch wenigstens zweimaliges Anzüchten und/oder Fermentieren in Verdünnungsreihen und anaeroben Kultivierungsbedingungen gewonnen wird.

7. Verfahren nach Anspruch 6, **dadurch gekennzeichnet, daß** die Anzucht und/oder Fermentation in einer Gasatmosphäre aus H₂ und CO₂ vorgenommen wird.

8. Verfahren nach Anspruch 7, **dadurch gekennzeichnet, daß** die Gasatmosphäre mit einem Verhältnis H₂:CO₂ von 10:90 bis 90:10, insbesondere etwa 80:20, gewählt wird.

9. Verfahren nach einem der Ansprüche 6 bis 8, **dadurch gekennzeichnet, daß** die Anzucht und/oder Fermentation bei einem Überdruck von 0,2 bis 3 bar, insbesondere 0,5 bis 1 bar, durchgeführt wird.

10. Verfahren nach einem der Ansprüche 6 bis 9, **dadurch gekennzeichnet, daß** die Anzucht bzw. Fermentation bei einer Temperatur von 35 bis 42 °C, insbesondere etwa 37 °C, durchgeführt wird.

11. Verfahren nach einem der Ansprüche 6 bis 10, **dadurch gekennzeichnet, daß** die Anzucht und/oder Fermentation bei einem pH-Wert zwischen 6 und 8, insbesondere zwischen 7 und 7,5, durchgeführt wird.

12. Verfahren nach einem der Ansprüche 6 bis 11, **dadurch gekennzeichnet, daß** die Anzucht und/oder Fermentation in einer Medienbereitung, enthaltend Komponenten gewählt aus: Arginin, Citrulin, Pepton, Hefeextrakt, Fettsäuremischung (en) , Minerallösung(en), Glucose, Häminlösung, Menadion, Vitaminlösung, Spurenelementen, Reduktionsmittel, durchgeführt wird.

13. Verfahren nach einem der Ansprüche 6 bis 12, **dadurch gekennzeichnet, daß** zu Beginn der Anzucht und/oder Fermentation 0,1 bis 0,5 Gew.-%, insbesondere 0,2 Gew.-%, Glucose zugesetzt werden.

14. Verfahren nach einem der Ansprüche 6 bis 13, **dadurch gekennzeichnet, daß** Enzympräparationen des aktiven, Trichothecen-detoxifizierenden Mikroorganismus und/oder andere anaerobe Mikroorganismen zugesetzt werden.

15. Verfahren nach einem der Ansprüche 6 bis 14, **dadurch gekennzeichnet, daß** aus der Anzucht- und/oder Fermentationslösung die Reinkultur des Mikroorganismus DSM 11798 durch wenigstens zwei weitere Verdünnungsreihen in der Medienbereitung, insbesondere unter Zusatz von L-Arginin als Wachstumsstimulator, gewonnen wird.

16. Verfahren nach Anspruch 15, **dadurch gekennzeichnet, daß** zur Fermentation der Reinkultur der Medienbereitung 1 bis 4 Gew.-% eines Reduktionsmittels, insbesondere einer Mischung von Cystein/Natriumsulfid/Natriumcarbonatlösung, zugesetzt wird.

17. Verfahren nach einem der Ansprüche 6 bis 16, **dadurch gekennzeichnet, daß** die Anzucht- und/oder Fermentationslösung durch Konzentrieren, insbesondere Zentrifugieren oder Filtrieren und/oder Stabilisieren, insbesondere durch Gefrier- oder Sprühtrocknen oder Encapsulieren, aufgearbeitet wird.

18. Verfahren nach Anspruch 17, **dadurch gekennzeichnet, daß** das Stabilisieren unter Zusatz eines Füllstoffes oder Trägermaterials, wie Aluminiumsilikaten, Kieselguren, Kohlehydraten, Zuckeralkoholen, Stärken, Milch und Molkenpulver, Proteinhydrolysaten, Hefen, PVPP durchgeführt wird.

19. Verwendung des Mikroorganismus in Reinkultur (DSM 11798) zur Herstellung eines Futtermittelzusatzes.

20. Verwendung nach Anspruch 19, **dadurch gekennzeichnet, daß** die Reinkultur (DSM 11798) als gefrier- bzw. sprühgetrocknetes und/oder encapsuliertes Immobilisat, gegebenenfalls unter Zusatz eines Trägermaterials, eingesetzt wird.

21. Futtermittelzusatz zur Inaktivierung von Trichothecenen in Futtermitteln bzw. im Verdauungstrakt von Tieren, **dadurch gekennzeichnet, daß** der Futtermittelzusatz eine Reinkultur eines Mikroorganismus nach einem der Ansprüche 1 bis 5 bzw. einen Mikroorganismus, hergestellt nach einem der Ansprüche 6 bis 18, in einer Menge von 10⁵ bis 10¹² Zellen/kg, insbesondere 10⁷ bis 10⁹ Zellen/kg, pro 1000 kg Futtermittel enthält.

22. Futtermittelzusatz nach Anspruch 21, **dadurch gekennzeichnet, daß** zusätzlich ein Trägermaterial und/oder Füllstoff in einer Menge von 0,5 bis 8 kg/1000 kg, insbesondere 0,7 bis 4 kg/1000 kg, des Futtermittels enthalten ist.

23. Futtermittelzusatz nach Anspruch 22, **dadurch gekennzeichnet, daß** als Trägermaterial bzw. Füllstoff Aluminiumsilikate, Kieselgure, Kohlehydrate, Zuckeralkohole, Stärke, Milch und Molkenpulver, Proteinhydrolysate, Hefen und/oder PVPP enthalten sind.

24. Futtermittelzusatz nach Anspruch 21, 22 oder 23, **dadurch gekennzeichnet, daß** der Futtermittelzusatz aus einer Mischung aus 1 bis 65 Gew.-%, insbesondere 5 bis 50 Gew.-%, des sprüh- bzw. gefriergetrockneten Immobilates des Mikroorganismus und 99 bis 35 Gew.-%, insbesondere 95 bis 50 Gew.-%, Trägermaterial und/oder Füllstoff besteht.

25. Verwendung eines Futtermittelzusatzes nach einem der Ansprüche 21 bis 24 zur Inaktivierung von Deoxynivalenol (DON), T-2 Toxin, HT-2 Toxin, Nivalenol, Monoacetoxyscirpenol, Diacetoxyscirpenol, Trichodermol, Verrucarin, Rorodin, Acetyl-Deoxynivalenol, Isotrichodermin, Hydroxyisotrichodermin, Calonectrin, T-2 Tetraol, T-2 Triol, Deacetylneosolaniol, Neosolaniol, Acetylneosolaniol, Sporotrichiol, Trichotriol, Sambucinol und Culmorin in einem Futtermittel bzw. dem Verdauungstrakt von Tieren.

## Claims

1. Microorganism of the genus Eubacterium in pure culture, DSM 11798, for the detoxification of trichothecenes by reductive cleaving of the 12,13-epoxy ring thereof.

2. Microorganism according to claim 1, **characterized in that** it detoxifies deoxynivalenol (DON), T-2 toxin, HT-2 toxin, nivalenol, monoacetoxyscirpenol, diacetoxyscirpenol, trichodermol, verrucarin, rorodin, acetyldeoxynivalenol, isotrichodermin, hydroxyisotrichodermin, calonectrin, T-2 tetraol, T-2 triol, deacetylneosolaniol, neosolaniol, acetylneosolaniol, sporotrichiol, trichotriol, sambucinol and culmorin.

3. Microorganism according to claim 1 or 2, **characterized in that** the microorganism detoxifies the trichothecenes by reductive biotransformation of the epoxide group which is contained in the molecule.

4. Microorganism according to claim 1, 2 or 3, **characterized in that** it is an anaerobic grampositive, rod-shaped, non-spore-forming bacterium which occurs individually, in pairs or in long chains.

5. Microorganism according to one of claims 1 to 4, **characterized in that** it contains a 16S RNA sequence, namely

6. Process for obtaining a culture of the microorganism according to one of claims 1 to 5, **characterized in that** a mixed culture DSM 11799 is first obtained from the microorganism and Enterococcus casseliflavus from bovine rumen by culturing and/or fermenting at least twice in dilution series and anaerobic culturing conditions.

7. Process according to claim 6, **characterized in that** the culturing and/or fermentation is carried out in a gas atmosphere of H₂ and CO₂.

8. Process according to claim 7, **characterized in that** the gas atmosphere is selected with a ratio of H₂:CO₂ of 10:90 to 90:10, in particular of approximately 80:20.

9. Process according to one of claims 6 to 8, **characterized in that** the culturing and/or fermentation is carried out at an overpressure of 0.2 to 3 bar, in particular 0.5 to 1 bar.

10. Process according to one of claims 6 to 9, **characterized in that** the culturing or fermentation is carried out at a temperature of 35 to 42 °C, in particular 37 °C.

11. Process according to one of claims 6 to 10, **characterized in that** the culturing and/or fermentation is carried out at a pH between 6 and 8, in particular at a pH between 7 and 7.5.

12. Process according to one of claims 6 to 11, **characterized in that** the culturing and/or fermentation is carried out in a media preparation comprising components selected from amongst: arginine, citrulline, peptone, yeast extract, fatty acid mixture(s), mineral solution(s), glucose, haemin solution, menadione, vitamin solution, trace elements, reducing agents.

13. Process according to one of claims 6 to 12, **characterized in that** 0.1 to 0.5 % by weight, in particular 0,2 % by weight, of glucose is added at the start of the culturing and/or fermentation.

14. Process according to one of claims 6 to 13, **characterized in that** enzyme preparations of the active, trichothecene-detoxifying microorganism and/or other anaerobic microorganisms are added.

15. Process according to one of claims 6 to 14, **characterized in that** the pure culture of the microorganism DSM 11798 is obtained from the culturing and/or fermentation solution by at least two further dilution series in the media preparation, in particular adding L arginine as a growth stimulator.

16. Process according to claim 15, **characterized in that**, for the fermentation of the pure culture of the media preparation, 1 to 4 % by weight of a reducing agent in particular a mixture of cysteine/sodium sulphide/sodium carbonate in solution, is added.

17. Process according to one of claims 6 to 16, **characterized in that** the culturing and/or fermentation solution is worked up by concentrating, in particular fermentation solution is concentrated by centrifuging or filtering and/or stabilizing, in particular by freeze-drying or spray-drying or encapsulating.

18. Process according to claim 17, **characterized in that** the stabilizing is carried out with addition of a filler or carrier material, such as aluminium silicates, kieselguhrs, carbohydrates, sugar alcohols, starches, milk and whey powder, protein hydrolysates, yeasts, PVPP.

19. Use of the microorganism in pure culture (DSM 11798) for the preparation of a feedstuff additive.

20. Use according to claim 19, **characterized in that** the pure culture (DSM 11798) is employed as a freeze- or spray-dried and/or encapsulated immobilizate, potentially by adding a carrier material.

21. Feedstuff additive for the inactivation of trichothecenes in feedstuffs or in the digestive tract of animals, **characterized in that** the feedstuff additive contains a pure culture of a microorganism according to one of claims 1 to 5 or a microorganism prepared according to one of claims 6 to 18, in an amount from 10⁵ to 10¹² cells/kg per 1000 kg, in particular 10⁷ to 10⁹ cells/kg, of feedstuff.

22. Feedstuff additive according to claim 21, **characterized in that** it additionally contains a carrier material and/or filler in an amount from 0.5 to 8 kg/1000 kg, in particular 0.7 to 4 kg/1000 kg, of the feedstuff.

23. Feedstuff additive according to claim 22, **characterized in that** aluminium silicates, kieselguhrs, carbohydrates, sugar alcohols, starch, milk and whey powder, protein hydrolysates, yeasts and/or PVPP are contained as a carrier material or filler.

24. Feedstuff additive according to claim 21, 22 or 23, **characterized in that** the feedstuff additive consists of a mixture of 1 to 65 % by weight, in particular 5 to 50 % by weight, of the spray- or freeze-dried immobilizate of the microorganism and 99 to 35% by weight, in particular 95 to 50 % by weight, of carrier material and/or filler.

25. Use of a feedstuff additive according to one of claims 21 to 24 for the inactivation of deoxynivalenol (DON), T-2 toxin, HT-2 toxin, nivalenol, monoacetoxyscirpenol, diacetoxyscirpenol, trichodermol, verrucarin, rorodin, acetyldeoxynivalenol, isotrichodermin, hydroxyisotrichodermin, calonectrin, T-2 tetraol, T-2 triol, deacetylneosolaniol, neosolaniol, acetylneosolaniol, sporotrichiol, trichotriol, sambucinol and culmorin in a feedstuff or the digestive tract of animals.

## Revendications

1. Micro-organisme du genre Eubacterium en culture pure, DSM 11798, approprié à la détoxification des trichothécènes par clivage réducteur du cycle 12,13-époxy de ceux-ci.

2. Micro-organisme selon la revendication 1, **caractérisé en ce qu'**il détoxifie le désoxynivalénol (DON), la toxine T-2, la toxine HT-2, le nivalénol, le monoacétoxyscirpénol, le diacétoxyscirpénol, le trichodermol, la verrucarine, la rorodine, l'acétyldésoxynivalénol, l'isotrichodermine, l'hydroxyisotrichodermine, la calonectrine, le tétraol T-2, le triol T-2, le désacétylnéosolaniol, le néosolaniol, l'acétylnéosolaniol, le sporotrichiol, le trichotriol, le sambucinol, la culmorine.

3. Micro-organisme selon la revendication 1 ou 2, **caractérisé en ce que** le micro-organisme détoxifie les trichothécènes par biotransformation réductrice du groupe époxyde présent dans la molécule.

4. Micro-organisme selon la revendication 1, 2 ou 3, **caractérisé en ce qu'**il est une bactérie anaérobie à Gram positif, en forme de brindille, non sporogène, qui se présente individuellement, par paires ou bien en chaînes longues.

5. Micro-organisme selon l'une des revendications 1 à 4, **caractérisé en ce qu'**il présente une séquence d'ARN 16S, soit

6. Procédé d'obtention d'une culture du micro-organisme selon l'une des revendications 1 à 5, **caractérisé en ce qu'**une culture mixte DSM 11799, constituée du micro-organisme et de Enterococcus casseliflavus est d'abord obtenue à partir de panses de boeuf par au moins une double culture et/ou fermentation en séries de dilution et dans des conditions de culture anaérobies.

7. Procédé selon la revendication 6, **caractérisé en ce que** la culture et/ou fermentation est opérée dans une atmosphère gazeuse de H₂ et de CO₂.

8. Procédé selon la revendication 7, **caractérisé en ce que** l'atmosphère gazeuse est choisie avec un rapport H₂/CO₂ de 10/90 à 90/10, en particulier environ 80/20.

9. Procédé selon l'une des revendications 6 à 8, **caractérisé en ce que** la culture et/ou fermentation est réalisée à une surpression de 0,2 à 3 bars, en particulier 0,5 à 1 bar.

10. Procédé selon l'une des revendications 6 à 9, **caractérisé en ce que** la culture ou fermentation se fait à une température de 38 à 42°C, en particulier à environ 37°C.

11. Procédé selon l'une des revendications 6 à 10, **caractérisé en ce que** la culture et/ou fermentation se fait à un pH situé entre 6 et 8, en particulier entre 7 et 7,5.

12. Procédé selon l'une des revendications 6 à 11, **caractérisé en ce que** la culture et/ou fermentation est réalisée dans une préparation de milieux, comprenant des composants choisis parmi : l'arginine, la citrulline, la peptone, l'extrait de levure, un ou des mélanges d'acides gras, une ou des solutions minérales, le glucose, la solution d'hémine, la ménadione, la solution vitaminée, les éléments traces, les agents réducteurs.

13. Procédé selon l'une des revendications 6 à 12, **caractérisé en ce que**, au début de la culture et/ou de la fermentation, 0,1 à 0,5 % en poids, en particulier 0,2 % en poids, de glucose est ajouté.

14. Procédé selon l'une des revendications 6 à 13, **caractérisé en ce que** des préparations enzymatiques du micro-organisme actif détoxifiant les trichothécènes et/ou d'autres micro-organismes anaérobies sont additionnées.

15. Procédé selon l'une des revendications 6 à 14, **caractérisé en ce que** l'on obtient la culture pure du micro-organisme DSM 11798 à partir de la solution de culture et/ou de fermentation par au moins deux autres séries de dilution dans la préparation de milieux, en particulier par l'addition de L-arginine à titre de stimulateur de croissance.

16. Procédé selon la revendication 15,**caractérisé en ce que**, pour la fermentation de la culture pure de la préparation de milieux, 1 à 4 % en poids d'un agent réducteur, en particulier d'un mélange de cystéine/sulfure de sodium/ solution de carbonate de sodium, sont additionnés.

17. Procédé selon l'une des revendications 6 à 16, **caractérisé en ce que** la solution de culture et/ou de fermentation est traitée par concentration, en particulier par centrifugation, ou filtration et/ou stabilisation, en particulier par lyophilisation ou séchage par pulvérisation ou encapsulation.

18. Procédé selon la revendication 17, **caractérisé en ce que** la stabilisation se fait par addition d'une charge ou d'un support, comme les aluminosilicates, les diatomées, les hydrates de carbone, les alcools de sucre, les amidons, le lait et la poudre de petit lait, les hydrolysats de protéines, les levures, la PVPP.

19. Utilisation du micro-organisme en culture pure (DSM 11798) pour la préparation d'un additif pour substances fourragères.

20. Utilisation selon la revendication 19, **caractérisé en ce que** la culture pure (DSM 11798) est utilisée sous forme d'immobilisat lyophilisé ou séché par pulvérisation et/ou encapsulé, le cas échéant en ajoutant un support.

21. Additif pour substances fourragères, destiné à l'inactivation des trichothécènes dans des substances fourragères ou bien dans le tractus digestif des animaux, **caractérisé en ce que** l'additif substances fourragères comprend une culture pure d'un micro-organisme selon l'une des revendications 1 à 5 ou un micro-organisme, préparé selon l'une des revendications 6 à 18, en une quantité de 10⁵ à 10¹² cellules/kg, en particulier 10⁷ à 10⁹ cellules/kg, pour 1000 kg de substances fourragères.

22. Additif pour substances fourragères selon la revendication 21, **caractérisé en ce qu'**un support et/ou une charge est présent en plus, en une quantité de 0,5 à 8 kg/1000 kg, en particulier 0,7 à 4 kg/1000 kg de substances fourragères.

23. Additif pour substances fourragères selon la revendication 22, **caractérisé en ce que** des aluminosilicates, des diatomées, des hydrates de carbone, des alcools de sucre, des amidons, du lait et de la poudre de petit lait, des hydrolysats de protéines, des levures et/ou de la PVPP sont présents à titre de support ou bien de charges.

24. Additif pour substances fourragères selon la revendication 21, 22 ou 23, **caractérisé en ce que** l'additif pour substances fourragères se compose d'un mélange de 1 à 65 % en poids, en particulier de 5 à 50 % en poids, de l'immobilisat séché par pulvérisation ou lyophilisé du micro-organisme et 99 à 35 % en poids, en particulier 95 à 50 % en poids de matière support et/ou de charge.

25. Utilisation d'un additif pour substances fourragères selon l'une des revendications 21 à 24 pour l'inactivation du désoxynivalénol (DON), de la toxine T-2, de la toxine HT-2, du nivalénol, monoacétoxyscirpénol, du diacétoxyscirpénol, du trichodermol, de la verrucarine, de la rorodine, de l'acétyl-désoxynivalénol, de l'isotrichodermine, de l'hydroxyisotrichodermine, de la calonectrine, du tétraol T-2, du triol T-2, du désacétyl-néosolaniol, du néosolaniol, de l'acétyl-néosolaniol, du sporotrichiol, du trichotriol, du sambucinol, de la culmorine dans une substance fourragère ou dans le tractus digestif d'animaux.
